## Europäisches Patentamt

## European Patent Office

## Office européen des brevets

(19)

(11) Veröffentlichungsnummer: **0 162 005**
**B1**

(12) **EUROPÄISCHE PATENTSCHRIFT**

(45) Veröffentlichungstag der Patentschrift:
22.06.88

(51) Int. Cl.⁴: **A 61 F 2/34,** A 61 N 1/32

(21) Anmeldenummer: 85730059.4

(22) Anmeldetag: 13.04.85

(54) Einschraubpfanne für ein künstliches Hüftgelenk.

(30) Priorität: 13.04.84 DE 3414514

(43) Veröffentlichungstag der Anmeldung:
21.11.85 Patentblatt 85/47

(45) Bekanntmachung des Hinweises auf die Patenterteilung:
22.06.88 Patentblatt 88/25

(84) Benannte Vertragsstaaten:
AT CH DE FR LI

(56) Entgegenhaltungen:
DE - A - 3 240 592
DE - C - 2 315 517

(73) Patentinhaber: BIOTRONIK Mess- und Therapiegeräte GmbH & Co Ingenieurbüro Berlin, Sieversufer 8, D-1000 Berlin 47 (DE)

(72) Erfinder: Weigert, Manfred, Prof. Dr. med., Cimbernstrasse 22, D-1000 Berlin 38 (DE)
Erfinder: Werhahn, C., Dr. med., Bayernallee 43, D-1000 Berlin 19 (DE)

(74) Vertreter: Christiansen, Henning, Dipl.-Ing., CHRISTIANSEN & NINNEMANN Dietrich-Schäfer-Weg 21, D-1000 Berlin 41 (DE)

## Beschreibung

Die Erfindung betrifft eine Einschraubpfanne der im Oberbegriff des Anspruchs 1 angegebenen Art.

Aus der DE-C-2 315 517 ist eine Hüftgelenkprothese bekannt, welche elektrisch voneinander isolierte Elektroden im Schaftbereich aufweist, die im Fall einer Lockerung der Prothese ermöglichen, durch niederfrequente Wechselströme erneut die Bildung von Knochensubstanz anzuregen, um somit eine einwandfreie Verbindung mit dem Knochen wieder herzustellen.

Nachteilig ist bei der bekannten Prothese und ähnlichen Vorrichtungen zu Knochenstimulation mittels Stromzuführung über Elektroden, dass eine Anwendung für die Hüftpfanne nicht möglich ist und sich ausserdem durch die Anordnung der verschiedenen Elektroden im Schaftbereich nur eine zufällige, lokal stark unterschiedliche Stromverteilung erzielen lässt, so dass eine das Knochenwachstum gesteuert fördernde Stimulation für eine grössere Fläche nicht erreichbar ist. Weiterhin ist die Verwendung eines Stimulationsstroms mit wechselnder Polarität nicht günstig.

Der im Anspruch 1 angegebenen Erfindung liegt demgegenüber die Aufgabe zugrunde, eine Pfanne der eingangs angegebenen Gattung anzugeben, welche durch Stimulation des Knochenwachstums eine Fixierung der Pfanne derart unterstützt, dass ein fester Sitz schon nach verhältnismässig kurzer Zeit gesichert ist.

Die Erfindung beruht auf der Erkenntnis, dass mittels Gleichstromstimulation, bei der die implantierte Elektrode die Kathode und die Hautoberfläche die Anode bildet, über im wesentlichen die gesamte tragende Aussenoberfläche des Implantats ein Knochenwachstum erzielbar ist, welches ein sicheres Einwachsen bereits in der unmittelbar auf die Implantation folgenden Phase ermöglicht, wobei die Stimulation des Knochenbzw. Gewebewachstums bevorzugt auf – über die Gesamtfläche gleichmässig verteilte – kleinere Zonen oder Bereiche beschränkt wird, in denen jeweils die Bildung von Knochensubstanz eine maximale Auswirkung auf die Befestigung des Implantats hat.

Bei einer einschraubbaren Hüftpfanne war es bisher nicht möglich, einzelne Bereiche der Oberfläche als Elektrode auszubilden, da die gesamte mit dem Knochen in tragender Verbindung stehende Aussenoberfläche als Gewinde ausgebildet ist und somit die Tragflanken kaum die Möglichkeit bieten, dort Elektrodenoberflächen anzuheften oder in sonstiger Weise anzubringen.

Es wurde jedoch gefunden, dass ein stabiler Sitz einer Einschraubpfanne auch dann gewährleistet werden kann, wenn der Grund des Gewindes mit neu zu bildender Knochensubstanz ausgefüllt wird. Um ein leichtes Einschrauben und eine Gängigkeit eines Gewindes zu gewährleisten, wird die wendelförmige Nut tiefer geschnitten als sie von dem einzuschraubenden Gewinde ausgefüllt wird; zumal der Spitzenbereich der Gewindeflanken wegen des geringen tragenden Querschnitts ohnehin zur Kraftübertragung bei eingeschraubtem Gewinde nur wenig beiträgt. Dadurch, dass dieser das Einschrauben erleichternde Freibereich – in bevorzugter Weiterbildung der Erfindung – mit einem umlaufenden Draht versehen wird, kann erreicht werden, dass der nicht von Material ausgefüllte Spitzenbereich des Gewindes mit Knochensubstanz angefüllt wird und sich somit das Gewinde «verklemmt», so dass ein Herausschrauben bzw. schon ein ungewolltes Drehen um einen kleinen Winkel sicher verhindert ist. Dieser Mechanismus wirkt auch dann, wenn die Pfanne – bei konischer Ausbildung des Gewindes – nicht vollständig eingeschraubt ist, da in diesem Fall durch das angeregte Knochenwachstum auch eine Verfestigung des – an sich zu lockeren – Sitzes erreicht wird.

Bei anderen bevorzugten Weiterbildungen der Erfindung ist auch in vertikal verlaufenden – das Gewinde in Querrichtung durchziehenden – Nuten eine wirksame Elektrodenfläche vorgesehen, was gegebenenfalls durch entprechendes Verlegen des die Elektrode bildenden Drahtes innerhalb der Nuten erfolgen kann. Während der Grund des Gewindes der Einschraubpfanne keilförmig spitz ausgebildet ist, wird der nach aussen gerichtete Teil des Profils bevorzugt trapezförmig abgestumpft oder verrundet. Wird die Erzielung einer besonders hohen Festigkeit des Prothesensitzes durch Kallusbildung in möglichst kurzer Zeit gewünscht, so lassen sich in eine Einkerbung der in der Mantelfläche des äusseren Kegelstumpfes gelegenen oberen Seitenkanten des trapezförmigen Querschnitts zusätzlich zur stimulierenden Oberfläche beitragende Elektrodendrähte verlegen.

Dadurch wird ebenfalls zur Erhöhung der Festigkeit des Sitzes durch «Verklemmen» des Gewindes beigetragen. Da die hier dargestellte Stimulation des Knochenwachstums innerhalb eines Gewindes die Gewindereibung erhöht und damit eine Drehsicherung bewirkt, ist die erzielbare Verfestigung grösser als bei der Stimulation des Wachstums von Knochenbereichen, die einer Belastung durch Scher- oder Zugkräfte ausgesetzt sind. Durch das erfindungsgemäss erzielte Verklemmen wird die verfügbare Knochenoberfläche bei einem minimalen Volumen von zusätzlich durch angeregtes Wachstum vergrösserter Menge von Knochensubstanz in maximaler Weise verstärkt.

Gemäss anderen vorteilhaften Weiterbildungen der Erfindung sind die Kontaktelemente zur Stromeinleitung an verschiedenen Stellen unabhängig von der beim Einschrauben der Pfanne erreichten Position stets von der Eingriffsseite bei der Operation zugänglich. Dabei ist es besonders günstig, dass sich der die Elektrode bildende Draht durch die walzenförmige Ausbildung eines der Anschlussterminals über eine Rotationsbewegung der ihn aufnehmenden Aussparung problemlos spannen lässt.

Das Ende des zylindrischen Stifts ist dabei bevorzugt mit einem Schlitz oder einer entsprechenden Aussparung zu versehen, welche eine Drehmomentübertragung mittels eines Werkzeugs zulassen.

Vorteilhafte Weiterbildungen der Erfindung sind in den Unteransprüchen gekennzeichnet bzw. werden bei der nachstehenden Darstellung zusammen mit einer bevorzugten Ausführung näher beschrieben.

Fig. 1 zeigt ein Ausführungsbeispiel der erfindungsgemässen Pfanne in einer Ansicht von unten;

Fig. 2 zeigt die Pfanne insgesamt im Schnitt mit einem in

Fig. 2a dargestellten Detail, wobei die Schnittebene von Fig. 2 aus Fig. 1 ersichtlich ist;

Fig. 3 zeigt das Ausführungsbeispiel der erfindungsgemässen Pfanne gemäss Fig. 1 in einer Seitenansicht, wobei der Bereich eines Anschlussterminals für die Elektrodenzuführung im Schnitt dargestellt ist.

Die Erfindung soll nunmehr unter gleichzeitiger Bezugnahme auf die verschiedenen Figuren erläutert werden, wobei nach Bedarf jeweils auf die Figur, welche das betreffende Element am deutlichsten zeigt, verwiesen wird.

Der Körper 1 der Pfanne besteht aus einem körperverträglichen Kunststoff, welcher die für den angegebenen Zweck erforderliche Festigkeit aufweist. Er ist kegelstumpfförmig ausgebildet, wobei die beim Einschrauben voranzuführende Stirnfläche einen kleineren Durchmesser aufweist. Das Vorsehen eines Gewindes 2 ist dabei ebenso wie die innere kalottenförmige Aufnahme 3 für eine Gelenkkugel grundsätzlich bekannt. Weiterhin vorgesehen sind Werkzeugnuten 4, 5 und 6, die um jeweils 120° versetzt auf dem Umfang angeordnet sind und beim Eingriff eines Schraubwerkzeugs zur Drehmomentübertragung dienen.

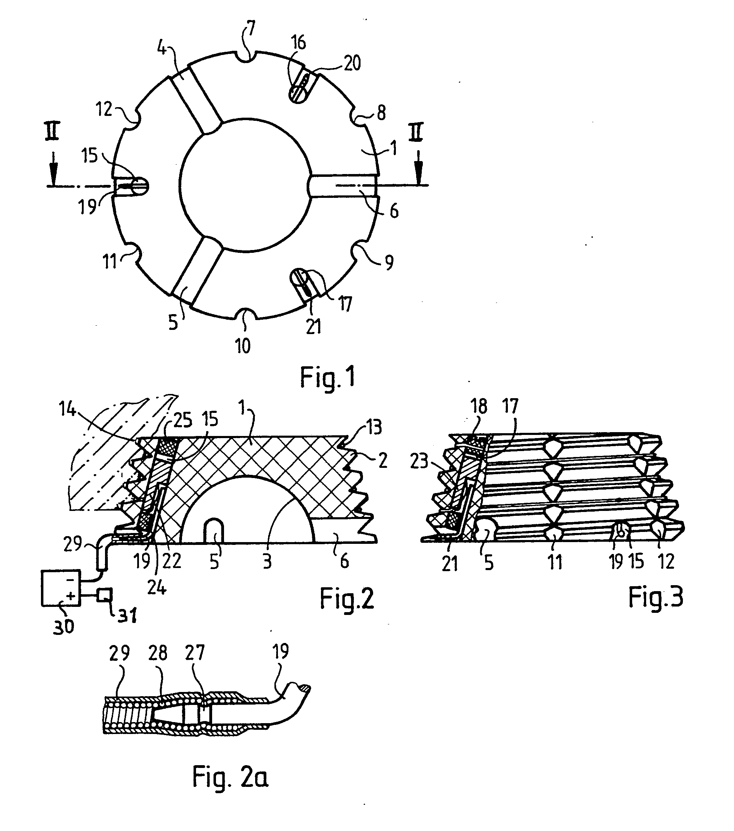

Nuten 7 bis 12, welche quer zum Gewinde verlaufen, dienen zur Sicherung der Pfanne gegen ungewolltes Ausdrehen in der ersten Phase nach der Operation, wobei die Nuten bei einer möglichen selbstschneidenden Ausführung zur Aufnahme der beim Eindrehen der Pfanne erzeugten Knochenspäne dienen. Die hier dargestellte Ausführung wird allerdings in ein vorgeschnittenes Gewinde eingesetzt, welches in der Form demjenigen an der Aussenoberfläche der Pfanne entspricht. Das Gewinde wird dabei so vorgeschnitten, dass die im Knochen erzeugten Aussparungen spitzwinklig keilförmig geformt sind, wobei zwischen den eingeschnittenen Nuten noch Material verbleibt. Das in den Knochen einzuschneidende Profil 14 ist in Fig. 2 für den linken Gewindebereich in der Zeichnung als Teilausschnitt dargestellt.

Im Grunde des Gewindes verläuft eine Drahtwendel 13 als Elektrode, welche die Stimulationselektrode bildet. Diese Wendel verbindet drei Anschlussterminals, die mit 15, 16 und 17 bezeichnet sind. Sämtliche Terminals 15 bis 17 liegen elektrisch auf demselben Potential und können wahlweise – je nach Position der Pfanne im eingeschraubten Zustand – mit einem Anschluss kontaktiert werden, wie er in Fig. 2a vergrössert wiedergegeben ist. Während die Anschlussterminals 15 und 16 den Draht lediglich tangieren, der zu diesem Zweck durch eine bei dem Terminal angeordnete Bohrung zu dem entsprechenden zylinderförmigen Teil hingeführt wird, welches in einer entsprechenden Aussparung des Pfannenkörpers angeordnet ist, ist mit dem Terminal 17 auch das obere Ende des Elektrodendrahtes 13 verbunden, so dass mit dem Drehen des entsprechenden zylindrischen Elementes 18 durch Eingriff in eine schlitzförmige Aussparung sich der Draht von oben her spannen lässt, nachdem er bei der Montage zunächst nur lose aufgelegt wurde.

Die Aussparung, in die das Element 18 eingeführt wurde, ist derart bemessen, dass sich in den verschiedenen Stellungen, die beim Spannen erreicht werden, ein Klemmsitz ergibt. Kontaktierungselemente 19, 20 und 21 lassen sich von unten in die zylindrischen Kontaktelemente einschieben und werden darin durch federnde Kontaktschleifer 22 und 23 (Fig. 2 bzw. 3) und durch Führungsstücke 24 festgehalten. Elastische Stopfen (ein Stopfen 25 in Fig. 2 bildet dabei ein Beispiel) verschliessen jeweils die in der Pfanne vorhandenen Aussparungen nach dem Einsetzen der Terminalanschlüsse.

Der Durchmesser des die Elektrode bildenden Drahtes ist derart bemessen, dass sich unter Berücksichtigung der durch die Körperflüssigkeit gebildeten Elektrolyten im Körper bei der entsprechenden angelegten Spannung eine Stromdichte von ca. 4 mA/cm$^2$ – bezogen auf die Knochenfläche – ergibt.

Das in Fig. 2a vergrösserte Ende des Anschlussstiftes 19 zeigt, dass dieses konisch und spitz zulaufend ausgebildet ist und im Bereich des vollen Durchmessers eine umlaufende Einschnürung 27 aufweist, in welche sich die Zuleitungswendel der Elektrodenleitung unter der Zugspannung des die Wendel 28 umgebenden Schlauches 29 hineinzieht. Auf diese Weise ergibt sich ein Anschlusssystem, welches mit einfachen Mitteln sicher und zuverlässig arbeitet und ausserdem noch eine Abdichtung gegenüber der Körperflüssigkeit sicherstellt. Die Elektrodenzuleitung kann innerhalb der Operationsphase ohne besonderen Aufwand hergestellt und gegebenenfalls verändert werden.

Eine Gleichstromquelle 30 ist mit einem Anschlussterminal der Pfanne 1 verbunden, deren Elektrodenflächen die Kathode bilden, während der Gegenpol durch eine Hautanode 31 gebildet wird.

Aus Fig. 2 ist ersichtlich, dass der Elektrodendraht in einem Hohlraum liegt, in dem er beim Eingriff des konischen Gewindes der Pfanne in das in den Knochen eingeschnittene Gewebe verbleibt. Gerade das in diesem Hohlraum angeregte Knochenwachstum sorgt – wie eingangs dargestellt – für eine optimale Verfestigung des Pfannensitzes, da hier in einem Bereich Knochengewebewachstum stimuliert wird, welcher einerseits die Gängigkeit des Gewindes beim Einschrauben sichert, andererseits, nachdem er von Knochengewebe ausgefüllt ist, sicherstellt, dass sich das Gewinde nicht mehr verdrehen lässt.

Bei anderen – in der Zeichnung nicht dargestellten – Ausführungsformen kann der Draht auch durch die Vertikalnuten 7 bis 12 geführt werden, so dass durch das hier gebildete Knochengewebe eine zusätzliche Sperre gegen Verdrehen erzeugt wird.

Eine Knochengewebestimulation in diesem Bereich ist aber im allgemeinen nicht notwendig, da das Wachstum wegen des grösseren auszufüllenden Hohlraums auch längere Zeit in Anspruch nehmen würde. Weiterhin besteht die Möglichkeit, noch Nuten in den erhabenen Teilen des Gewindes der Pfanne vorzusehen, in die Drähte eingelegt werden können, so dass auch der entsprechende, auf der Seite des Knochengewebes in der Spitze des dort einzuschneidenden Gewindes, verlaufende Bereich mit durch stimuliertes Wachstum erzeugtem Knochenmaterial angefüllt werden kann.

Die Erfindung beschränkt sich in ihrer Ausführung nicht auf das vorstehend angegebene Beispiel. Vielmehr sind eine Vielzahl von Varianten denkbar, welche von der dargestellten Lösung auch bei grundsätzlich anders gearteten Ausführungen Gebrauch machen.

**Patentansprüche**

1. Einschraubpfanne für ein künstliches Hüftgelenk, dadurch gekennzeichnet, dass in vertieften Teilen des Gewindes (14) leitende Bereiche (13) vorgesehen sind, die mit einer Gleichspannungsquelle (30) zur Knochenstimulation in leitender Verbindung stehen.

2. Einschraubpfanne nach Anspruch 1, dadurch gekennzeichnet, dass die leitenden Bereiche durch einen Draht (13) gebildet werden, welcher entlang des Gewindegrundes unter Vorspannung geführt ist.

3. Einschraubpfanne nach Anspruch 2, dadurch gekennzeichnet, dass mindestens ein zylindrischer Stift (15 bis 17) vorgesehen ist, welcher in die Pfanne (1) mit Klemmsitz eingelassen ist und zum Spannen des Drahtes eines seiner Enden bzw. einen Mittelabschnitt in mehrfacher Umschlingung auf seiner Aussenoberfläche hält.

4. Einschraubpfanne nach einem der vorangehenden Ansprüche, dadurch gekennzeichnet, dass der zylindrische Stift (15 bis 17) in jeweils eine Ausnehmung der Pfanne (1) eingefügt ist und an einem seiner Enden eine durch einen Durchbruch des Pfannenkörpers erreichbare Ausnehmung für einen Drehmomentanschluss aufweist.

5. Einschraubpfanne nach einem der vorangehenden Ansprüche, dadurch gekennzeichnet, dass die Pfanne im Bereich des Gewindes (13) vertikal verlaufende Nuten (7 bis 12) aufweist.

6. Einschraubpfanne nach Anspruch 5, dadurch gekennzeichnet, dass mindestens im Bereich des Grundes der Nuten ebenfalls leitende Zonen vorgesehen sind.

7. Einschraubpfanne nach einem der vorangehenden Ansprüche, dadurch gekennzeichnet, dass ein vertikal geführter Draht vorgesehen ist.

8. Einschraubpfanne nach einem der vorangehenden Ansprüche, dadurch gekennzeichnet, dass mit dem zylindrischen Stift ein Kontaktelement (19) als Anschluss für eine Zuleitung verbunden ist.

9. Einschraubpfanne nach einem der vorangehenden Ansprüche, dadurch gekennzeichnet, dass auf dem Umfang der Pfanne verteilt mehrere den umlaufenden Draht (13) kontaktierende zylindrische Stifte (15 bis 17) vorgesehen sind, welche jeweils einen Anschluss (19 bis 21) für die Zuleitung aufweisen.

10. Einschraubpfanne nach einem der vorangehenden Ansprüche, dadurch gekennzeichnet, dass die Anschlüsse (19 bis 21) jeweils um 120° versetzt auf dem Aussenumfang der Pfanne (1) im – in Einschraubrichtung gesehen – rückwärtigen Bereich angeordnet sind.

11. Einschraubpfanne nach einem der vorangehenden Ansprüche, dadurch gekennzeichnet, dass die Anschlüsse (19 bis 21) an ihren Enden zugespitzt ausgebildet sind und eine umlaufende Nut (27) aufweisen, in welche sich die elastischen Wendeln (28) der mit einem elastischen Isoliermantel (29) versehenen Zuleitung einlagern und somit eine Sicherung gegen unbeabsichtigtes Lösen bilden.

12. Stimulationsanordnung für eine Einschraubpfanne nach einem der vorangehenden Ansprüche, dadurch gekennzeichnet, dass die Einschraubpfanne die Kathode bildet, während die Anode auf der Hautoberfläche vorgesehen ist.

**Claims**

1. A screw-in cup for an artificial hip joint, characterized in that conductive regions (13) are provided in recessed parts of the thread (14), which regions are in conductive connection with a d.c. voltage supply (30) for bone stimulation.

2. A screw-in cup according to claim 1, characterized in that the conducting regions are formed by a wire (13) which is guided along the thread base under pretension.

3. A screw-in cup according to claim 2, characterized in that at least one cylindrical pin (15 to 17) is provided which is let into the cup (1) with clamp fit and for tensioning the wire holds one of its ends or a middle section in multiple coils on its outer surface.

4. A screw-in cup according to any one of the preceding claims, characterized in that the cylindrical pin (15 to 17) is inserted, in each case, in a recess of the cup (1) and has, at one of its ends, a recess for a torque connection which can be reached through an opening of the cup body.

5. A screw-in cup according to any one of the preceding claims, characterized in that the cup has vertically extending grooves (7 to 12) in the region of the thread (13).

6. A screw-in cup according to claim 5, characterized in that conducting zones are also provided at least in the region of the grooves.

7. A screw-in cup according to any one of the preceding claims, characterized in that a vertically guided wire is provided.

8. A screw-in cup according to any one of the preceding claims, characterized in that a contact element (19), as connection for a lead wire, is connected to the cylindrical pin.

9. A screw-in cup according to any one of the preceding claims, characterized in that a plurality of cylindrical pins (15 to 17) contacting the encircling wire (13) are provided distributed on the periphery of the cup, which pins, in each case, have a connection (19 to 21) for the lead wire.

10. A screw-in cup according to any one of the preceding claims, characterized in that the connections (19 to 21) are arranged in each case displaced by 120 degrees on the outer periphery of the cup (1) in the rearward region-as seen in the screw-in direction.

11. A screw-in cup according to any one of the preceding claims, characterized in that the connections (19 to 21) are made pointed at their ends and have an encircling groove (27) in which the elastic coils (28) of the lead wire provided with an elastic insulating sheathing (29) are encased and thus form a protection against unintentional loosening.

12. A stimulation arrangement for a screw-in cup according to any one of the preceding claims, characterized in that the screw-in cup forms the cathode, while the anode is provided on the skin's outer surface.

**Revendications**

1. Cupule à fixer en place par vissage, pour une articulation artificielle de la hanche, caractérisée en ce que des régions conductrices (13) sont prévues dans des parties approfondies du filetage (14) et sont en liaison conductrice avec une source de tension continue (30) pour la stimulation de l'os.

2. Cupule selon la revendication 1, caractérisée en ce que les régions conductrices sont formées par un fil métallique (13) qui est guidé sous précontrainte le long du fond du filet.

3. Cupule selon la revendication 2, caractérisée en ce qu'elle comprend au moins une tige cylindrique (15 à 17) qui est encastrée à ajustement serré dans la cupule (1) et qui, pour tendre le fil, maintient plusieurs spires d'une extrémité ou d'une partie médiane du fil sur sa surface extérieure.

4. Cupule selon une des revendications précédentes, caractérisée en ce que la tige ou chaque tige cylindrique (15 à 17) est insérée dans un évidement de la cupule (1) et est pourvue à l'une de ses extrémités d'un évidement que l'on peut atteindre à travers une percée du corps de la cupule et qui sert à l'application d'un couple pour tourner la tige.

5. Cupule selon une des revendications précédentes, caractérisée en ce qu'elle présente des rainures (7 à 12) orientées verticalement dans la région du filetage (13).

6. Cupule selon la revendication 5, caractérisée en ce que des zones conductrices sont prévues également dans au moins la région du fond des rainures.

7. Cupule selon une des revendications précédentes, caractérisée par la prévision d'un fil guidé verticalement.

8. Cupule selon une des revendications précédentes, caractérisée en ce qu'un élément de contact (19) servant de borne pour une connexion est relié à la tige cylindrique.

9. Cupule selon une des revendications précédentes, caractérisée par la prévision de plusieurs tiges cylindriques (15 à 17) réparties sur la périphérie de la cupule et en contact avec le fil (13), faisant le tour de la cupule, tiges qui présentent chacune une borne (19 à 21) pour la connexion.

10. Cupule selon une des revendications précédentes, caractérisée en ce que les bornes (19 à 21) sont disposées sur la périphérie extérieure de la cupule (1) avec un décalage mutuel de 120° et dans la région arrière dans le sens de l'enfoncement de la cupule, par vissage, dans l'os.

11. Cupule selon une des revendications précédentes, caractérisée en ce que les bornes (19 à 21) sont effilées à leurs extrémités et présentent une gorge circonférentielle (27), dans laquelle vient se loger l'une au moins des spires élastiques (28) de la connexion, pourvue d'une gaine isolante élastique (29), de manière à empêcher ainsi le détachement involontaire.

12. Dispositif de stimulation pour une cupule à vis selon une des revendications précédentes, caractérisé en ce que la cupule forme la cathode, tandis que l'anode est prévue à la surface de la peau.

0162005

Fig. 1

Fig.2

Fig.3

Fig. 2a

7